# EUROPEAN PATENT SPECIFICATION

(11) **EP 3 684 341 B1**
(45) Date of publication and mention of the grant of the patent: **08.04.2026**
(21) Application number: 18840094.9
(22) Date of filing: 19.09.2018
(51) Int. Cl.: A61K 9/16, A61K 9/24, A61K 9/20, A61K 31/683, A61K 31/536, A61K 31/675, A61K 31/7068

(54) **PHARMACEUTICAL COMBINATIONS COMPRISING TENOFOVIR, EMTRICITABINE AND EFAVIRENZ**
PHARMAZEUTISCHE KOMBINATIONEN AUS TENOFOVIR, EMTRICITABIN UND EFAVIRENZ
COMBINAISONS PHARMACEUTIQUES DE TÉNOFOVIR, D'EMTRICITABINE ET D'ÉFAVIRENZ

(30) Priority: 20.09.2017 TR 201713954
(43) Date of publication of application: 29.07.2020
(73) Proprietor: Sanovel Ilac Sanayi ve Ticaret A.S., Sisli - Istanbul (TR)
(72) Inventor: YILDIRIM, Ediz, 34398 Istanbul (TR); BAS, Oguz, 34460 Istanbul (TR); MURATOGLU, Yesim, 34460 Istanbul (TR); DEMIR, Bülent, 34460 Istanbul (TR); ATMACA, Ibrahim, 34460 Istanbul (TR); TÜRKYILMAZ, Ali, 34460 Istanbul (TR)
(74) Representative: Genç Ilhan, Oznur
(86) International application number: PCT/TR2018/050508
(87) International publication number: WO 2019/059868

(56) References cited:
- WO-A2-2006/135932
- WO-A2-2006/135933
- WO-A2-2008/096369
- FDA: "Tablet Scoring: Nomenclature, Labeling, and Data for Evaluation", GUIDANCE FOR INDUSTRY, 1 March 2013 (2013-03-01), pages 1 - 5, XP055619619, Retrieved from the Internet <URL:https://www.fda.gov/media/81626/download> [retrieved on 20190909]
- JACQUES EMMANUEL REGINALD ET AL: "Tablet Scoring: Current Practice, Fundamentals, and Knowledge Gaps", APPLIED SCIENCES, vol. 9, no. 15, 29 July 2019 (2019-07-29), pages 3066, XP055869525, Retrieved from the Internet <URL:https://www.mdpi.com/2076-3417/9/15/3066/pdf> DOI: 10.3390/app9153066
- VERRUE CHARLOTTE ET AL: "Tablet-splitting: a common yet not so innocent practice : Tablet-splitting", JOURNAL OF ADVANCED NURSING, vol. 67, no. 1, 12 December 2010 (2010-12-12), GB, pages 26 - 32, XP055869610, ISSN: 0309-2402, DOI: 10.1111/j.1365-2648.2010.05477.x
- ANONYMOUS: "Atripla EPAR Scientific Discussion", 18 December 2007 (2007-12-18), London, pages 1 - 48, XP002789411, Retrieved from the Internet <URL:https://www.ema.europa.eu/en/documents/scientific-discussion/atripla-epar-scientific-discussion_en.pdf> [retrieved on 20190304]

## Description

### Field of Invention

The present invention relates to pharmaceutical combinations comprising tenofovir or a pharmaceutically acceptable salt thereof, emtricitabine or a pharmaceutically acceptable salt thereof and efavirenz or a pharmaceutically acceptable salt thereof.

### Background of Invention

Tenofovir is a nucleotide analogue reverse transcriptase inhibitor (NtRTI). The chemical name of tenofovir is ({[(2R)-1-(6-amino-9H-purin-9-yl)propan-2-yl]oxy}methyl)phosphonic acid and has the structure shown in the following formula I.

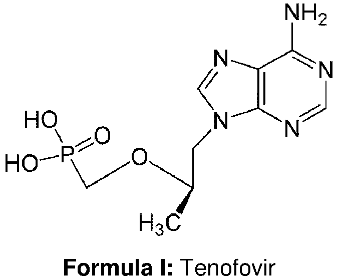

Tenofovir disoproxil fumarate is a prodrug of tenofovir and is marketed under the brand name VIREAD^{®}. Tenofovir disoproxil fumarate blocks the reverse transcriptase enzyme which is crucial for the human immunodeficiency virus 1 (HIV-1) and the hepatitis B virus. Therefore, it is used in the treatment of HIV-1 and hepatitis B. Tenofovir disoproxil fumarate has the following structural formula.

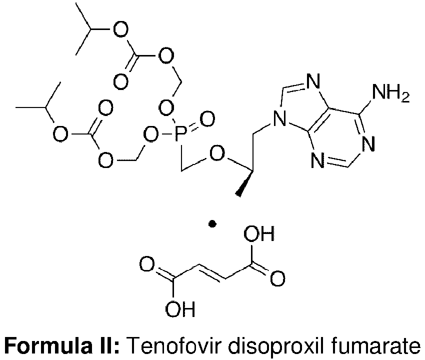

Emtricitabine is a synthetic fluoro derivative of thiacytidine with potent antiviral activity. The chemical name of emtricitabine is 4-amino-5-fluoro-1-[(2R,5S)-2-(hydroxymethyl)-1,3-oxathiolan-5-yl]-1,2-dihydropyrimidin-2-one and has the structure shown in the following formula III.

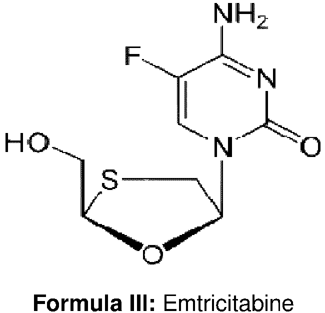

Emtricitabine, with trade name EMTRIVA^{®}, is a nucleoside reverse transcriptase inhibitor (NRTI) for the treatment of HIV infection in adults and children.

Emtricitabine is phosphorylated to form emtricitabine 5'-triphosphate within the cell. This metabolite inhibits the activity of human immunodeficiency virus (HIV) reverse transcriptase both by competing with the natural substrate deoxycytidine 5'-triphosphate and by incorporation into viral DNA causing a termination of DNA chain elongation.

Efavirenz is a synthetic non-nucleoside reverse transcriptase inhibitor (NNRTI) with antiviral activity. Efavirenz binds directly to the human immunodeficiency virus type 1 (HIV-1) RT, an RNA-dependent DNA polymerase, blocking its function in viral DNA replication.

Efavirenz, which is used as part of highly active antiretroviral therapy (HAART) for the treatment of a human immunodeficiency virus (HIV) type 1, is marketed under the brand name SUSTIVA^{®}

Its chemical name is (4S)-6-chloro-4-(2-cyclopropylethynyl)-4-(trifluoromethyl)-1H-3,1-benzoxazin-2-one and it has the structure shown in the following formula IV.

WO 2006/135932 A1 discloses a triple combination comprising efavirenz, emtricitabine and tenofovir. Tablet size and swalling difficulty are one of the concerns in WO 2006/135932 A1. As a solution, a dry granulated composition of emtricitabine and tenofovir is disclosed. This composition essentially retains the crystalline APIs and is substantially free of dried eutectic emtricitabine /tenofovir DF. Said dry granulated composition is then mixed with emtricitabine. Dry granulated compositions include the direct product of dry granulation, as well as products made from such granules including tablets, capsules, suppositories and other pharmaceutical dosage forms.

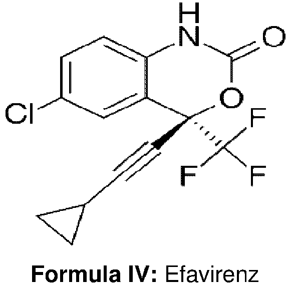

The use of combinations of compounds can yield an equivalent antiviral effect with reduced toxicity, or an increase in drug efficacy. The decrease in dosages thanks to the administration of combinations can reduce the frequency of occurrence of drug-resistant variants of HIV.

The prior art has many formulation examples of binary combinations of tenofovir disoproxil fumarate and emtricitabine for the treatment of HIV infection. It is also suggested to add a third antiviral active agent to this combination. This active agent can be a protease inhibitor (PI), a nucleoside reverse transcriptase inhibitor (NRTI), a non-nucleoside reverse transcriptase inhibitor (NNRTI) or an integrase inhibitor are some of them.

According to an approach in the art, all the compounds may be administered in a binary or ternary combinations at once. In another administration example, these compounds are delivered serially, sequentially, parallelly or simultaneously in separate pharmaceutical formulations or combinations. This administration, which is named as alternation therapy, is a little risky due to the possible delay, omission or misadministration of a second or a third compound.

Accordingly, it is more preferable to formulate a single dosage form comprising all the compounds. Therefore, overcoming the incompatibility problem of these compounds and providing the chemical stability of their combinations play an important role in the formulation success. Another important point to take into consideration is to maintain patient compliance, since the size of the dosage form enlarges due to the increase in the number of required layer to ensure the stability. Hence, one of the significant problems with multilayer tablets is the swallowing difficulty.

Patent document numbered EP1583542B1 basically emphasizes binary combinations of tenofovir disoproxil fumarate (tenofovir DF) and emtricitabine and aims to develop chemically stable tablet formulations of them. The document makes mention of a treatment in which tenofovir disoproxil fumarate, emtricitabine, and efavirenz are administered by two separate tablets. It is also mentioned the possibility to make a unitary and chemically stable combination of these three compounds. However, this statement is left unsupported, since there is no suggestions or examples for such a ternary combination formulation.

The main problem with combining these three active agents into a unitary and homogeneous composition is the incompatibilities among them. The prior art gives the teaching that tenofovir and its pharmaceutically acceptable salts are incompatible with efavirenz or its formulations. In some documents in the art, this incompatibility is assumed as a problem related not to efavirenz but to the excipients, such as surfactants, which are present in the efavirenz formulations. Consequently; surfactants, particularly SLS, are excluded from tenofovir DF and emtricitabine combinations.

The technical problem handled by the prior art is the stability issues of tenofovir DF and emtricitabine binary combinations. Accordingly, dry granulation is suggested to enhance the stability and improvements related to the preparing process have been carried out.

Although ternary combinations with efavirenz is mentioned in the prior art, these statements don't touch on the increased tablet size caused by the bilayer form and its disadvantages such as swallowing difficulty. Therefore, the prior art does not give any teaching, suggestion or motivation about how to enhance patient compliance and ensure high stability and high dissolution rate at the same time.

Besides, in the prior art, the amount of binder and lubricant are always kept high in the formulations to maintain the stability. But it is a well-known fact that the increase in the amount of binder causes the decrease in the dissolution rate. Besides, the tablet size shall get bigger in such case. On the other hand, high amounts of lubricant, especially magnesium stearate, decreases dissolution rate and crushing strength and may also increase tablet friability *(*Handbook of Pharmaceutical Excipients, Sixth edition, page 405*).*

Obviously, there is still a need for a bilayer ternary combination of these active agents which has high stability and at the same time high strength and low friability to enable the administration of a scored tablet form which will enhance the patient compliance.

### Objects and Brief Description of The Invention

The main object of the present invention is to obtain pharmaceutical combinations comprising tenofovir disoproxil fumarate (tenofovir DF), emtricitabine and efavirenz eliminating all aforesaid problems and bringing additional advantages to the relevant prior art.

Another object of the present invention is to obtain combinations with high stability and patient compliance.

A further object of the present invention is to develop combinations having improved dissolution rate.

A further object of the present invention is to develop combinations having decreased friability and brittleness.

A further object of the present invention is to develop combinations having increased crushing strength.

Another object of the present invention is to provide a bilayer tablet dosage form which has surfactant in both layers.

Another object of the present invention is to provide a bilayer tablet dosage form which has decreased amount of binder in both layers.

Another object of the present invention is to provide a bilayer tablet dosage form which has decreased amount of lubricant in both layers.

Another object of the present invention is to provide a bilayer tablet dosage form with a score which is enabling serial, sequent and simultaneous administration of two smaller pieces of this dosage form.

Yet, another object of the present invention is to provide a bilayer tablet dosage form comprising at least one film coating to protect the pharmaceutical composition against the moisture to maintain the stability.

A further object of the present invention is to improve a process for preparing a bilayer tablet dosage form in which wet granulation is carried out for both layers.

### Detailed Description of Invention

In accordance with the objects outlined above, detailed features of the present invention are given herein.

### List of figures:

**Figure 1** shows the top view of an embodiment of the oral dosage form subjected to the invention which has one score.
**Figure 2** shows the lateral view of the embodiment of the oral dosage form subjected to the invention which has one score.
**Figure 3** shows the lateral cross sectional area of the embodiment of the oral dosage form subjected to the invention which has one score.
**Figure 4** shows the lateral view of another embodiment of the oral dosage form subjected to the invention which has two scores.
**Figure 5** shows the lateral cross sectional area of the embodiment of the oral dosage form subjected to the invention which has two score.

The present invention relates a scored tablet composition comprising
- a first component (1) comprising tenofovir or a pharmaceutically acceptable salt thereof and emtricitabine or a pharmaceutically acceptable salt thereof and
- a second component (2) comprising efavirenz or a pharmaceutically acceptable salt thereof;
wherein the composition is a solid dosage form which has two scores (3) placed bilaterally such that said scores provides the split of the tablet in half; wherein said first component (1) further comprises hydroxypropyl cellulose in an amount of between 0.1-10% by weight of the first component (1).

According to the preferred embodiment, the composition comprises;
- a first component (1) comprising tenofovir or a pharmaceutically acceptable salt thereof, emtricitabine or a pharmaceutically acceptable salt thereof and
- a second component (2) comprising efavirenz or a pharmaceutically acceptable salt thereof.

According to this preferred embodiment, these two components are present discretely in a single dosage form and more preferably these components are layers. According to the preferred embodiment, the first component (1) and the second component (2) are in contact with one another. In another embodiment, these components can be separated by an inert layer.

According to this preferred embodiment, the composition is a solid dosage form which is in the form of coated tablet, trilayer tablet, bilayer tablet, multilayer tablet, orally disintegrating tablet, mini tablet, buccal tablet, sublingual tablet, effervescent tablet, immediate release tablet, modified release tablet, gastric disintegrating tablet, or tablet in tablet.

According to the preferred embodiment the composition is in the form of a bilayer tablet.

According to this preferred embodiment, the dosage form has two scores (3) placed bilaterally such that said scores provides the split of the tablet in half, thus serial, sequent and simultaneous administration of at least two smaller pieces than the whole dosage form.

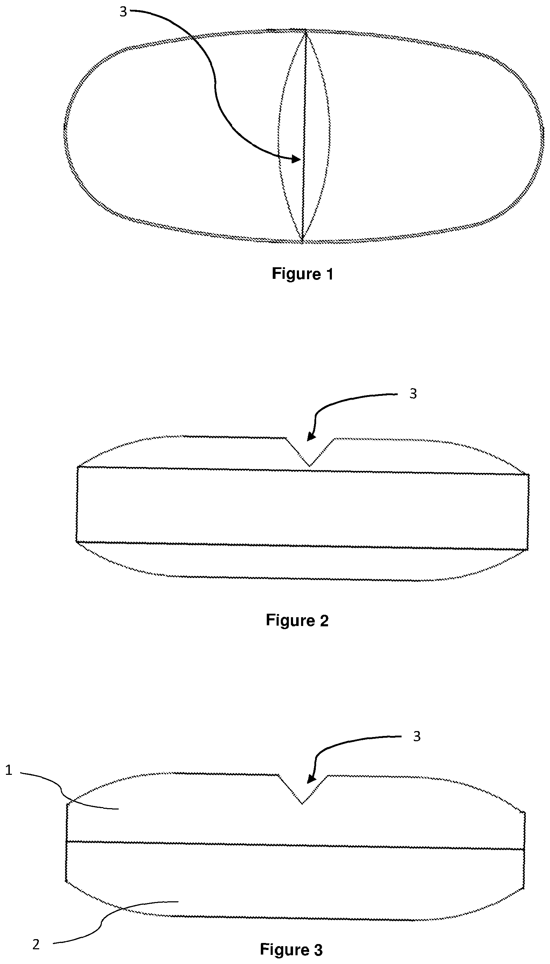

A dosage form which is scored on one side as seen in Figure 1, 2 and 3. (Reference)

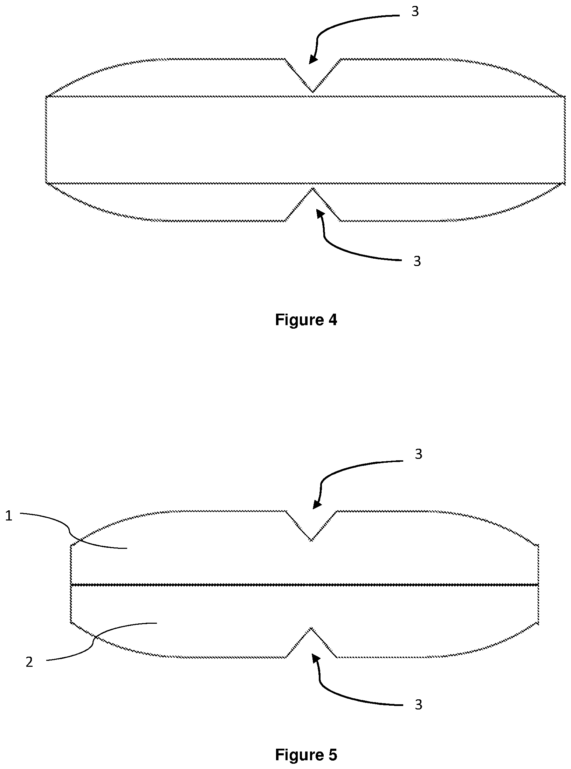

According to an embodiment, the dosage form is scored bilaterally as seen in Figure 4 and 5.

According to the preferred embodiment, the dosage form further comprises a film coating.

According to the most preferred embodiment of the invention; the composition comprises tenofovir disoproxil fumarate (tenofovir DF) with emtricitabine in the first component (1) and efavirenz in the second component (2).

The composition comprises tenofovir DF in the amount of 1-60% by weight of the total composition. Preferably this amount is between 5-50% by weight of the total composition. More preferably tenofovir DF is present in the amount of 10-30% by weight in the total composition.

The composition comprises emtricitabine in the amount of 1-60% by weight of the total composition. Preferably this amount is between 5-30% by weight of the total composition. More preferably emtricitabine is present in the amount of 5-20% by weight in the total composition.

The composition comprises efavirenz in the amount of 5-70% by weight of the total composition. Preferably this amount is between 10-60% by weight of the total composition. More preferably efavirenz is present in the amount of 30-50% by weight in the total composition.

In one embodiment of the invention, the weight of the total composition is between 500 and 2500 mg, preferably lower than 1595 mg.

The weight of the first layer (1) is between 150-1400 mg, preferably 300-1250 mg, more preferably 600-950 mg. In the most preferred embodiment, it is 750 mg.

The weight of the second layer (2) is between 150-1400 mg, preferably 300-1250 mg, more preferably 600-950 mg. In the most preferred embodiment, it is 800 mg.

Tenofovir DF is present in an amount of 100 to 1000 mg, preferably 200 to 500 mg and more preferably 300 mg in the composition.

Emtricitabine is present in an amount of 100 to 1000 mg, preferably 100 to 500 mg and more preferably 200 mg in the composition.

Efavirenz is present in an amount of 100 to 1000 mg, preferably 300 to 800 mg and more preferably 600 mg in the composition.

The amount of tenofovir DF is between 10-80%, preferably 20-70%, more preferably 30-50% by weight of the first component (1).

The amount of emtricitabine is between 5-70%, preferably 10-50%, more preferably 15-35% by weight of the first component (1).

According to the preferred embodiments, the first component (1) comprises hydroxypropyl cellulose in an amount of between 0.1-10% by weight of the first component (1) and at least one excipient selected from surfactants, solubilizers, wetting agents or mixtures thereof. According to the most preferred embodiment, the first component (1) comprises at least one surfactant which acts also as a wetting agent and a solubilizer and which is selected from the group comprising carboxylates, sulfates, sulfonates, phosphate esters.

In the preferred embodiment of the invention, the said surfactant is selected from alkyl sulfates. More preferably, the selected surfactant is sodium lauryl sulfate (SLS).

SLS is known to reduce interfacial tension between the solid particles and the granulation liquid and to improve wetting and deaggregation of active agent particles. Thus, it enables a homogeneous mixture and accordingly enhances physical properties of the final dosage form.

In the preferred embodiment, the amount of sodium lauryl sulfate is lower than 1% by weight of the first component (1). More preferably, this amount is lower than 0.5%.

It has been seen that the presence of this amount of SLS in the first component (1) surprisingly increases the final tablet hardness, strength and dissolution rate. Besides, it has been found that the timing of the addition of SLS in the first component (1) mixture is also important. The said advantages of SLS is more potent when it is added to the granules just before the final blending step of the mixture related to first component (1).

Improved hardness and strength of the tablet is significantly essential for this invention since the dosage form comprising the composition is preferably in the form of a scored tablet which is supposed to be split in half and this kind of tablets should have the strength not to crumble to more than two pieces. Improvement in powder wettability can often improve the bioavailability of the formulation.

According to this preferred embodiment, the first component (1) further comprises at least one excipient selected from binders, disintegrants, lubricants, glidants or mixtures thereof.

According to one embodiment of the invention, the first component (1) of the composition comprises hydroxypropyl cellulose (HPC) in an amount of between 0.1-10% by weight of the first component (1), and optionally at least one binder which is selected from the group comprising polyvinylpyrrolidone (PVP, povidone), microcrystalline cellulose, copovidone, copolyvidone, polyvinylpyrrolidone K30 (povidone K30), carnauba wax, pullulan, polymethacrylate, glyceryl behenate, carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, collagen, agar, pectin, hyaluronic acid, carbomer, cellulose acetate phthalate, hydroxyethyl methyl cellulose, poloxamer, polyethylene glycol (PEG), sugars, glucose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment, the amount of binder in the first component (1) is lower than 10% by weight of the first component (1). More preferably, this amount is lower than 5%. This value range, which is much lower than the prior art, is important to enhance the final tablet dissolution rate since it is known that high amounts of binder have a negative influence on dissolution rate.

According to preferred embodiments above, the first component (1) comprises hydroxypropyl cellulose as binder. More preferably, Klucel LF, which has a low viscosity (75-150 cps), is used as hydroxypropyl cellulose. The amount of hydroxypropyl cellulose is between 0.1-10%, preferably 1-5% by weight of the first component (1).

It has been seen that this selected binder and its amount in the formulation surprisingly enhances the dissolution profile.

According to one embodiment of the invention, the first component (1) of the composition comprises at least one diluent which is selected from the group comprising lactose monohydrate, lactose, microcrystalline cellulose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the first component (1) comprises lactose monohydrate as diluent. The amount of lactose monohydrate is between 1-50%, preferably 5-40%, more preferably 5-20% by weight of the first component (1).

According to one embodiment of the invention, the first component (1) of the composition comprises at least one disintegrant which is hydroxypropyl cellulose (HPC), and optionally further is selected from the group comprising croscarmellose sodium, sodium starch glycolate, microcrystalline cellulose, sodium carbonate, cross-linked polyvinylpyrrolidone (crospovidone), copovidon, polycarbophil, low-substitute poloxamer, alginic acid and alginates, ion-exchange resins, magnesium aluminum silica, sodium carboxy methyl cellulose, carboxy methyl cellulose calcium, docusate sodium, guar gum, polyacrylin potassium, sodium alginate, sodium glycine carbonate or mixtures thereof.

In the preferred embodiment, the first component (1) comprises two disintegrants which are microcrystalline cellulose and croscarmellose sodium. The amount of microcrystalline cellulose is between 0.1-50%, preferably 1-20%, more preferably 3-10% by weight of the first component (1). The amount of croscarmellose sodium is between 1-50%, preferably 5-30%, more preferably 8-20% by weight of the first component (1).

According to one embodiment of the invention, the first component (1) of the composition comprises at least one lubricant and at least one glidant which are selected from the group comprising sodium lauryl sulfate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

In the preferred embodiment of the invention, the first component (1) comprises magnesium stearate as lubricant and colloidal silicon dioxide as glidant.

According to the most preferred embodiment, the amount of magnesium stearate is lower than 2% by weight of the first component (1). More preferably, this amount is kept lower than 1.5% to reduce the final tablet friability and brittleness which is essential for scored tablets.

The amount of colloidal silicon dioxide is lower than 2% by weight of the first component (1). More preferably, this amount is lower than 1%. This amount of colloidal silicon dioxide is required to prevent sedimentation during the compression of scored tablets.

The amount of the first component (1) is between 10-90%, preferably 20-80%, more preferably 40-60% by weight of the total composition.

The amount of efavirenz is between 10-95%, preferably 30-90%, more preferably 60-80% by weight of the second component (2).

According to any of these embodiments, the second component (2) further comprises at least one excipient selected from binders, disintegrants, lubricants, glidants, surfactants, solubilizers, wetting agents or mixtures thereof.

According to one embodiment of the invention, the second component (2) of the composition comprises at least one binder which is selected from the group comprising polyvinylpyrrolidone (PVP, povidone), microcrystalline cellulose, copovidone, copolyvidone, polyvinylpyrrolidone K30 (povidone K30), carnauba wax, pullulan, polymethacrylate, glyceryl behenate, hydroxypropyl cellulose (HPC), carboxymethyl cellulose (CMC), methyl cellulose (MC), hydroxyethyl cellulose, sodium carboxymethyl cellulose (Na CMC), ethyl cellulose, polymetacrylates, polyethylene oxide, polyvinyl alcohol, polycarbophil, polyvinyl acetate and its copolymers, gelatin, xanthan gum, guar gum, alginate, carrageen, collagen, agar, pectin, hyaluronic acid, carbomer, cellulose acetate phthalate, hydroxyethyl methyl cellulose, poloxamer, polyethylene glycol (PEG), sugars, glucose syrups, natural gums, tragacanth gum, polyacrylamide, aluminum hydroxide, benthonite, laponite, setostearyl alcohol, polyoxyethylene-alkyl ethers, acacia mucilage, polydextrose or mixtures thereof.

According to the preferred embodiment, the amount of binder in the second component (2) is lower than 15% by weight of the second component (2). More preferably, this amount is lower than 5%. This value range, which is much lower than the prior art, is important to enhance the final tablet dissolution rate since it is known that high amounts of binder have a negative influence on dissolution rate.

According to preferred embodiments above, the second component (2) comprises hydroxypropyl cellulose as binder. More preferably, Klucel LF, which has a low viscosity (75-150 cps), is used as hydroxypropyl cellulose. The amount of hydroxypropyl cellulose is between 0.1-10%, preferably 1-5% by weight of the second component (2).

It has been seen that this selected binder and its amount in the formulation surprisingly enhances the dissolution profile.

According to one embodiment of the invention, the second component (2) of the composition comprises at least one diluent which is selected from the group comprising lactose monohydrate, lactose, microcrystalline cellulose, mannitol, spray-dried mannitol, starch, dextrose, sucrose, fructose, maltose, sorbitol, xylitol, inositol, kaolin, inorganic salts, polysaccharides, sodium chloride, dextrates, lactitol, maltodextrin, sucrose-maltodextrin mixture, trehalose, sodium carbonate, sodium bicarbonate, calcium carbonate or mixtures thereof.

According to the preferred embodiment of the invention, the second component (2) comprises lactose monohydrate as diluent. The amount of lactose monohydrate is between 1-50%, preferably 3-40%, more preferably 5-20% by weight of the second component (2).

According to one embodiment of the invention, the second component (2) of the composition comprises at least one disintegrant which is selected from the group comprising croscarmellose sodium, sodium starch glycolate, microcrystalline cellulose, sodium carbonate, hydroxylpropyl cellulose (HPC), cross-linked polyvinylpyrrolidone (crospovidone), copovidon, polycarbophil, low-substitute poloxamer, alginic acid and alginates, ion-exchange resins, magnesium aluminum silica, sodium carboxy methyl cellulose, carboxy methyl cellulose calcium, docusate sodium, guar gum, polyacrylin potassium, sodium alginate, sodium glycine carbonate or mixtures thereof.

In the preferred embodiment, the second component (2) comprises two disintegrants which are microcrystalline cellulose and croscarmellose sodium. The amount of microcrystalline cellulose is between 0.1-50%, preferably 1-20%, more preferably 2-10% by weight of the second component (2). The amount of croscarmellose sodium is between 0.5-50%, preferably 1-30%, more preferably 2-10% by weight of the second component (2).

According to one embodiment of the invention, the second component (2) of the composition comprises at least one lubricant and at least one glidant which are selected from the group comprising sodium lauryl sulfate, sodium stearyl fumarate, magnesium stearate, colloidal silicon dioxide, zinc stearate, calcium stearate, mineral oil, talc, polyethylene glycol, glyceryl monostearate, glyceryl palmitostearate, magnesium lauryl sulfate, fumaric acid, zinc stearate, stearic acid, hydrogenated natural oils, silica, paraffin or mixtures thereof.

In the preferred embodiment of the invention, the second component (2) comprises magnesium stearate as lubricant and colloidal silicon dioxide as glidant.

According to the most preferred embodiment, the amount of magnesium stearate is lower than 2% by weight of the second component (2). More preferably, this amount is kept lower than 1.5% to reduce the final tablet friability and brittleness which is essential for scored tablets.

The amount of colloidal silicon dioxide is lower than 2% by weight of the second component (2). More preferably, this amount is lower than 1%. This amount of colloidal silicon dioxide is required to prevent sedimentation during the compression of scored tablets.

According to one embodiment of the invention, the second component (2) of the composition comprises at least one surfactant which acts also as a wetting agent and a solubilizer and which is selected from the group comprising carboxylates, sulfates, sulfonates, phosphate esters.

In the preferred embodiment of the invention, the second component (2) comprises a surfactant which is selected from alkyl sulfates. More preferably, the selected surfactant is sodium lauryl sulfate (SLS).

SLS is known to reduce interfacial tension between the solid particles and the granulation liquid and to improve wetting and deaggregation of active agent particles. Thus, it enables a homogeneous mixture and accordingly enhances physical properties of the final dosage form.

The amount of sodium lauryl sulfate is higher than 2% by weight of the second component (2). In the preferred embodiment, this amount is between 2-10%, more preferably 2-5%. It has been seen that the presence of this amount of SLS in the second component (2) surprisingly increases the final tablet hardness, strength and dissolution rate.

Improved hardness and strength of the tablet is significantly essential for this invention since the composition is in the form of a scored tablet which is supposed to be split in half and this kind of tablets should have the strength not to crumble to more than two pieces. Improvement in powder wettability can often improve the bioavailability of the formulation.

The amount of the second component (2) is between 10-90%, preferably 20-80%, more preferably 40-60% by weight of the total composition.

According to the preferred embodiment of the invention, the dosage form of the composition further comprises at least one film coating to protect the composition against the moisture to maintain the stability. Suitable coating ingredients are selected from the group comprising hypromellose, lactose monohydrate, hydroxypropyl cellulose, polyvinyl alcohol (PVA), ethyl cellulose, polyethylene glycol (PEG), talc, triethyl citrate, polyvinyl alcohol-polyethylene glycol copolymers (Kollicoat^{®} IR), ethyl cellulose dispersions (Surelease^{®}), polyvinylprolidone, polyvinylprolidone-vinyl acetate copolymer (PVP-VA), all kinds of Opadry^{®}, pigments, dyes, titanium dioxide, iron oxide or mixtures thereof.

According to one embodiment, the film coating comprises polyvinyl alcohol, polyethylene glycol, titanium dioxide, talc, pigment or mixtures thereof.

Preferably, the coating is Opadry II. The amount of the film coating is 1-10%, preferably %2-5 by weight of the total composition.

According to these embodiments, the composition comprises;
- 10-80% of tenofovir disoproxil fumarate,
- 5-70% of emtricitabine,
- 0.1-10% of hydroxypropyl cellulose,
- 1-50% of lactose monohydrate,
- 0.1-50% of microcrystalline cellulose,
- 1-50% of croscarmellose sodium,
- lower than 2% of magnesium stearate,
- lower than 2% of colloidal silicon dioxide,
- lower than 1% of sodium lauryl sulfate by weight of the first component (1);
- 10-95% of efavirenz,
- 0.1-10% of hydroxypropyl cellulose,
- 1-50% of lactose monohydrate
- 0.1-50% of microcrystalline cellulose,
- 0.5-50% of croscarmellose sodium,
- lower than 2% of magnesium stearate,
- lower than 2% of colloidal silicon dioxide,
- 2-10% of sodium lauryl sulfate by weight of the second component (2) and
- 1-10% film coating by weight of the total composition.

These selected ratios ensure the required effective doses for the treatment, enhance stability and dissolution profile and reduce friability and brittleness of the film coated bilayer tablet subjected to the invention.

According to all these embodiments, the below given formulations can be used in the scored tablet composition subjected to the invention. These examples are not limiting the scope of the present invention and should be considered under the light of the foregoing detailed disclosure.

### Example 1: Ratios by weight of the components

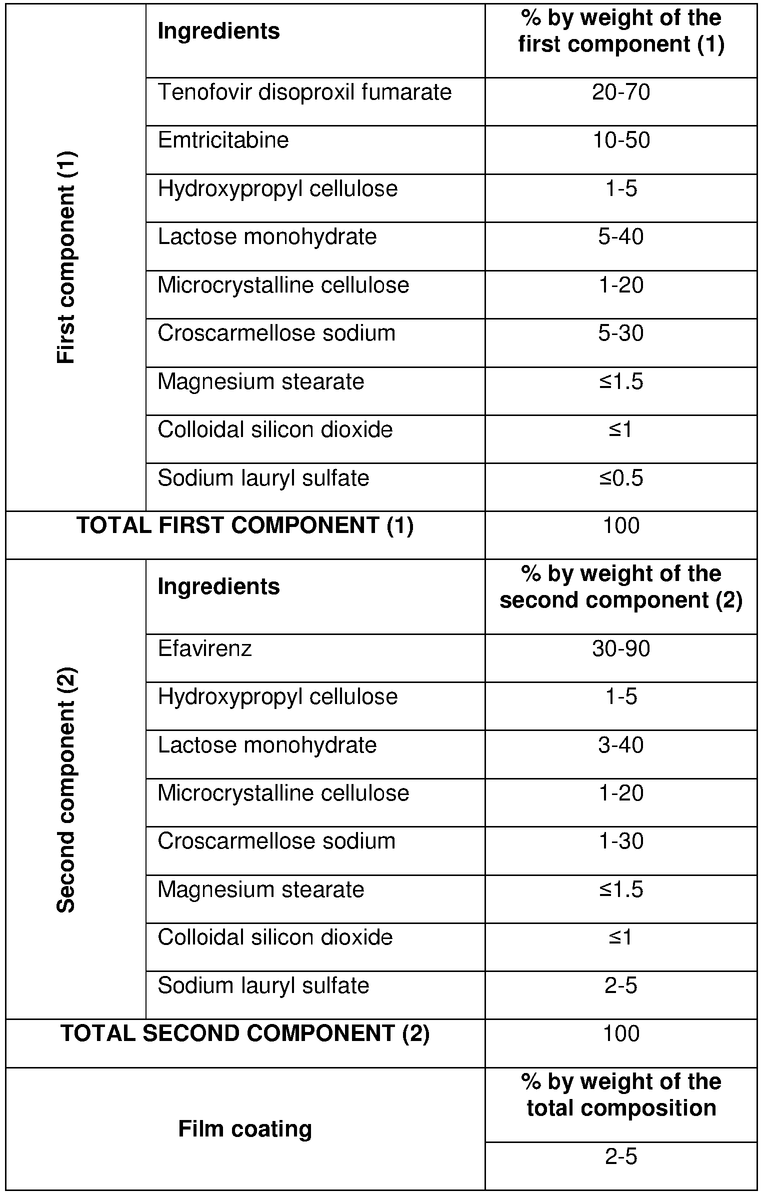

### Example 2: Ratios by weight of the total composition (coated bilayer tablet)

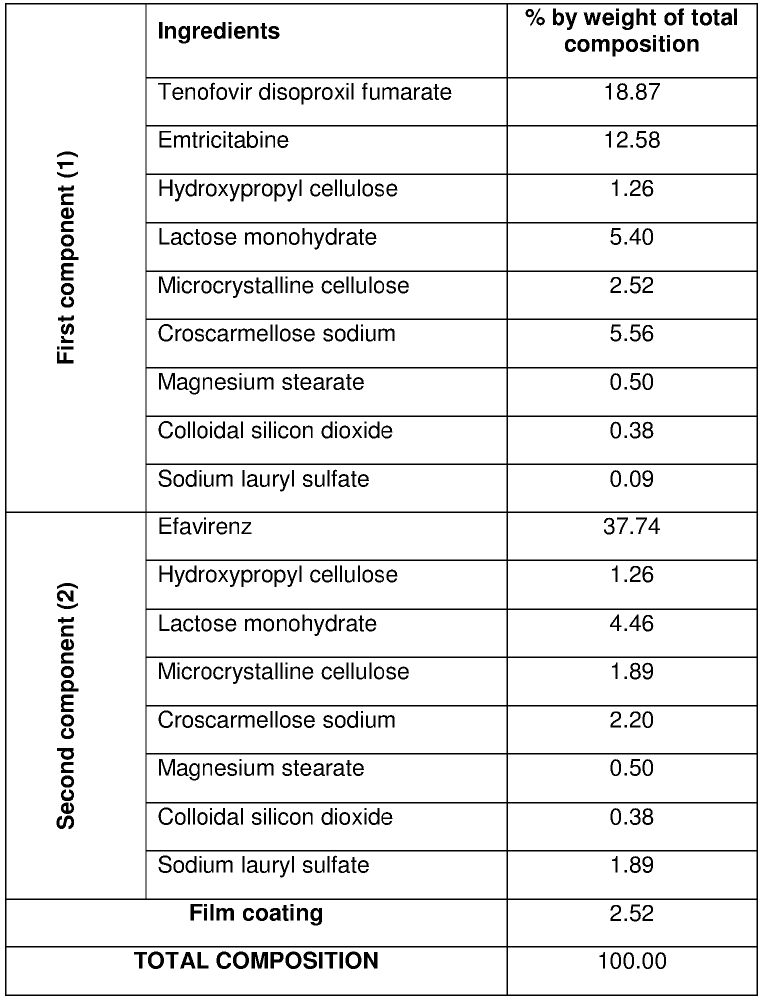

The compositions mentioned above are prepared by following these steps:
- preparation of the mixture composing the first component by (1) by wet granulation
- preparation of the mixture composing the second component (2) by wet granulation
- compressing these two mixtures together into scored bilayer tablets
- coating these scored bilayer tablets with a film coating solution

The mixture composing the first component (1) is prepared by following these steps:
- mixing tenofovir disoproxil fumarate, emtricitabine, microcrystalline cellulose, lactose monohydrate, hydroxypropyl cellulose and one-third of croscarmellose sodium
- granulating this mixture with water
- sieving the granules through a 5mm mesh and drying them
- sieving the dried granules again through a 1mm mesh
- adding two-thirds of croscarmellose sodium, sodium lauryl sulfate and colloidal silicon dioxide (sieved through the same 1mm mesh) to the sieved granules and mixing them for 15 minutes
- adding magnesium stearate (sieved through the same 1mm mesh) and mixing them for 5 minutes

The mixture composing the second component (2) is prepared by following these steps:
- mixing efavirenz, microcrystalline cellulose, lactose monohydrate, hydroxypropyl cellulose and croscarmellose sodium
- granulating this mixture with water and sodium lauryl sulfate
- sieving the granules through a 5mm mesh and drying them
- sieving the dried granules again through a 1mm mesh
- adding colloidal silicon dioxide (sieved through the same 1mm mesh) to the sieved granules and mixing them for 15 minutes
- adding magnesium stearate (sieved through the same 1mm mesh) and mixing them for 5 minutes

## Claims

1. A scored tablet composition comprising
- a first component (1) comprising tenofovir or a pharmaceutically acceptable salt thereof and emtricitabine or a pharmaceutically acceptable salt thereof and
- a second component (2) comprising efavirenz or a pharmaceutically acceptable salt thereof;
wherein the composition is a solid dosage form which has two scores (3) placed bilaterally such that said scores provides the split of the tablet in half ; wherein said first component (1) further comprises hydroxypropyl cellulose in an amount of between 0.1-10% by weight of the first component (1).

2. The scored tablet composition according to claim 1, wherein the first component (1) and the second component (2) are discrete layers.

3. The scored tablet composition according to claim 2, wherein the composition is in the form of a bilayer tablet.

4. The scored tablet composition according to claim 3, wherein the composition is in the form of a film-coated bilayer tablet.

5. The scored tablet composition according to any one of the preceding claims, wherein the said first component (1) comprises tenofovir disoproxil fumarate and emtricitabine and the said second component (2) comprises efavirenz.

6. The scored tablet composition according to any one of the preceding claims, wherein the first component (1) comprises a surfactant.

7. The scored tablet composition according to claim 6, wherein the surfactant is selected from the group comprising carboxylates, sulfates, sulfonates and phosphate esters.

8. The scored tablet composition according to claim 7, wherein the said surfactant is selected from sulfates which is sodium lauryl sulfate.

9. The scored tablet composition according to any one of the preceding claims, wherein the first component (1) further comprises at least one excipient selected from binders, disintegrants, lubricants, glidants or mixtures thereof.

10. The scored tablet composition according to any one of the preceding claims, wherein the second component (2) comprises at least one excipient selected from binders, disintegrants, lubricants, glidants, surfactants, solubilizers, wetting agents or mixtures thereof.

11. The scored tablet composition according to any one of the preceding claims, wherein the composition comprises;
- 10-80% of tenofovir disoproxil fumarate,
- 5-70% of emtricitabine,
- 0.1-10% of hydroxypropyl cellulose,
- 1-50% of lactose monohydrate,
- 0.1-50% of microcrystalline cellulose,
- 1-50% of croscarmellose sodium,
- lower than 2% of magnesium stearate,
- lower than 2% of colloidal silicon dioxide,
- lower than 1% of sodium lauryl sulfate by weight of the first component (1);
- 10-95% of efavirenz,
- 0.1-10% of hydroxypropyl cellulose,
- 1-50% of lactose monohydrate
- 0.1-50% of microcrystalline cellulose,
- 0.5-50% of croscarmellose sodium,
- lower than 2% of magnesium stearate,
- lower than 2% of colloidal silicon dioxide,
- 2-10% of sodium lauryl sulfate by weight of the second component (2) and
- 1-10% film coating by weight of the total composition.

12. A process for preparing the scored tablet composition according to claim 11, comprising the following steps:
- preparation of the mixture composing the first component by (1) by wet granulation
- preparation of the mixture composing the second component (2) by wet granulation
- compressing these two total mixtures together into scored bilayer tablets
- coating these scored bilayer tablets with a film coating solution

13. A process according to claim 12, wherein the said mixture composing the first component (1) is prepared by following these steps:
- mixing tenofovir disoproxil fumarate, emtricitabine, microcrystalline cellulose, lactose monohydrate, hydroxypropyl cellulose and one-third of croscarmellose sodium
- granulating this mixture with water
- sieving the granules and drying them
- sieving the dried granules again
- adding two-thirds of croscarmellose sodium, sodium lauryl sulfate and colloidal silicon dioxide to the sieved granules and mixing them
- adding magnesium stearate and mixing them

14. A process according to claim 13, wherein the said mixture composing the second component (2) is prepared by following these steps:
- mixing efavirenz, microcrystalline cellulose, lactose monohydrate, hydroxypropyl cellulose and croscarmellose sodium
- granulating this mixture with water and sodium lauryl sulfate
- sieving the granules and drying them
- sieving the dried granules again
- adding colloidal silicon dioxide to the sieved granules and mixing them
- adding magnesium stearate and mixing them

## Patentansprüche

1. Eine geteilte Tablettenzusammensetzung, umfassend
- eine erste Komponente (1) die Tenofovir oder ein pharmazeutisch akzeptables Salz davon und Emtricitabin oder ein pharmazeutisch akzeptables Salz davon umfasst und
- eine zweite Komponente (2) die Efavirenz oder ein pharmazeutisch verträgliches Salz davon umfasst;
wobei die Zusammensetzung eine feste Darreichungsform ist, die zwei Bruchkerben (3) aufweist, die so beidseitig angeordnet sind, dass die Tabletten durch diese Bruchkerben in zwei Hälften geteilt werden können; wobei die erste Komponente (1) ferner Hydroxypropylcellulose in einer Menge zwischen 0,1 - 10 Gew.-% der ersten Komponente (1) umfasst.

2. Die geteilte Tablettenzusammensetzung gemäß Anspruch 1, wobei die erste Komponente (1) und die zweite Komponente (2) getrennte Schichten sind.

3. Die geteilte Tablettenzusammensetzung gemäß Anspruch 2, wobei die Zusammensetzung in Form einer zweischichtigen Tablette vorliegt.

4. Die geteilte Tablettenzusammensetzung gemäß Anspruch 3, wobei die Zusammensetzung in Form einer filmbeschichteten Zweischichttablette vorliegt.

5. Die geteilte Tablettenzusammensetzung gemäß einem der vorstehenden Ansprüche, wobei die erste Komponente (1) Tenofovirdisoproxilfumarat und Emtricitabin umfasst und die zweite Komponente (2) Efavirenz umfasst.

6. Die geteilte Tablettenzusammensetzung gemäß einem der vorstehenden Ansprüche, wobei die erste Komponente (1) ein Tensid umfasst.

7. Die geteilte Tablettenzusammensetzung gemäß Anspruch 6, wobei das Tensid aus der Gruppe ausgewählt ist, die Carboxylate, Sulfate, Sulfonate und Phosphatester umfasst.

8. Die geteilte Tablettenzusammensetzung gemäß Anspruch 7, wobei das Tensid aus Sulfaten ausgewählt ist, die Natriumlaurylsulfat sind.

9. Die geteilte Tablettenzusammensetzung gemäß einem der vorstehenden Ansprüche, wobei die erste Komponente (1) ferner mindestens einen Hilfsstoff umfasst, der aus Bindemitteln, Sprengmitteln, Schmiermitteln, Gleitmitteln oder Mischungen davon ausgewählt ist.

10. Die geteilte Tablettenzusammensetzung gemäß einem der vorstehenden Ansprüche, wobei die zweite Komponente (2) mindestens einen Hilfsstoff umfasst, der aus Bindemitteln, Sprengmitteln, Schmiermitteln, Gleitmitteln, Tensiden, Lösungsvermittlern, Netzmitteln oder Mischungen davon ausgewählt ist.

11. Die geteilte Tablettenzusammensetzung gemäß einem der vorstehenden Ansprüche, wobei die Zusammensetzung umfasst:
- 10-80 % Tenofovirdisoproxilfumarat,
- 5-70 % Emtricitabin,
- 0,1-10 % Hydroxypropylcellulose,
- 1 - 50 % Lactose-Monohydrat,
- 0,1 - 50 % mikrokristalline Cellulose,
- 1-50 % Croscarmellose-Natrium,
- weniger als 2 % Magnesiumstearat,
- weniger als 2 % kolloidales Siliciumdioxid,
- weniger als 1 % Natriumlaurylsulfat,
bezogen auf das Gewicht der ersten Komponente (1);
- 10-95 % Efavirenz,
- 0,1-10 % Hydroxypropylcellulose,
- 1-50 % Lactose-Monohydrat
- 0,1-50 % mikrokristalline Cellulose,
- 0,5-50 % Croscarmellose-Natrium,
- weniger als 2 % Magnesiumstearat,
- weniger als 2 % kolloidales Siliciumdioxid,
- 2-10 % Natriumlaurylsulfat,
bezogen auf das Gewicht der zweiten Komponente (2) und
- 1-10 % Filmüberzug, bezogen auf das Gewicht der Gesamtzusammensetzung.

12. Verfahren zur Herstellung der Zusammensetzung für die geteilte Tablette gemäß Anspruch 11, umfassend die folgenden Schritte:
- Herstellung der Mischung, die die erste Komponente (1) bildet, durch Nassgranulierung
- Herstellung der Mischung, die die zweite Komponente (2) bildet, durch Nassgranulierung
- Zusammenpressen dieser beiden Gesamtmischungen zu geteilten Zweischichttabletten
- Beschichten dieser geteilten Zweischichttabletten mit einer Filmüberzugslösung

13. Verfahren gemäß Anspruch 12, wobei die Mischung, die die erste Komponente (1) bildet, durch die folgenden Schritte hergestellt wird:
- Mischen von Tenofovirdisoproxilfumarat, Emtricitabin, mikrokristalliner Cellulose, Lactose-Monohydrat, Hydroxypropylcellulose und einem Drittel Croscarmellose-Natrium
- Granulieren dieser Mischung mit Wasser
- Sieben der Granulate und Trocknen derselben
- erneutes Sieben des getrockneten Granulats Hinzufügen von zwei Dritteln Croscarmellose-Natrium, Natriumlaurylsulfat und kolloidalem Siliciumdioxid zu dem gesiebten Granulat und Mischen
- Zugabe von Magnesiumstearat und Vermischen

14. Verfahren nach Anspruch 13, wobei die Mischung, aus der die zweite Komponente (2) besteht, durch folgende Schritte hergestellt wird:
- Mischen von Efavirenz, mikrokristalliner Cellulose, Lactose-Monohydrat, Hydroxypropylcellulose und Croscarmellose-Natrium
- Granulieren dieser Mischung mit Wasser und Natriumlaurylsulfat
- Sieben der Granulate und Trocknen
- erneutes Sieben der getrockneten Granulate
- Zugabe von kolloidalem Siliciumdioxid zu den gesiebten Granulaten und Mischen
- Zugabe von Magnesiumstearat und Mischen

## Revendications

1. - Composition de comprimé sécable comprenant :
- un premier composant (1) comprenant du ténofovir ou un sel pharmaceutiquement acceptable de celui-ci et de l'emtricitabine ou un sel pharmaceutiquement acceptable de celle-ci ; et
- un second composant (2) comprenant de l'éfavirenz ou un sel pharmaceutiquement acceptable de celui-ci ;
dans laquelle la composition est une forme posologique solide qui présente deux rainures (3) placées de façon bilatérale de telle sorte que lesdites rainures permettent de diviser le comprimé en deux ; dans laquelle ledit premier composant (1) comprend en outre de l'hydroxypropyl cellulose dans une quantité d'entre 0,1 et 10 % en poids du premier composant (1).

2. - Composition de comprimé sécable selon la revendication 1, dans laquelle le premier composant (1) et le second composant (2) sont des couches discrètes.

3. - Composition de comprimé sécable selon la revendication 2, dans laquelle la composition se présente sous la forme d'un comprimé bicouche.

4. - Composition de comprimé sécable selon la revendication 3, dans laquelle la composition se présente sous la forme d'un comprimé bicouche pelliculé.

5. - Composition de comprimé sécable selon l'une quelconque des revendications précédentes, dans laquelle ledit premier composant (1) comprend du fumarate de ténofovir disoproxil et de l'emtricitabine et ledit second composant (2) comprend de l'éfavirenz.

6. - Composition de comprimé sécable selon l'une quelconque des revendications précédentes, dans laquelle le premier composant (1) comprend un tensioactif.

7. - Composition de comprimé sécable selon la revendication 6, dans laquelle le tensioactif est choisi dans le groupe comprenant les carboxylates, les sulfates, les sulfonates et les esters phosphates.

8. - Composition de comprimé sécable selon la revendication 7, dans laquelle ledit tensioactif est choisi parmi les sulfates, tel que le lauryl sulfate de sodium.

9. - Composition de comprimé sécable selon la revendication 8, dans laquelle le premier composant (1) comprend en outre au moins un excipient choisi parmi les liants, les désintégrants, les lubrifiants, les agents de glissement ou leurs mélanges.

10. - Composition de comprimé sécable selon l'une quelconque des revendications précédentes, dans laquelle le second composant (2) comprend au moins un excipient choisi parmi les liants, les désintégrants, les lubrifiants, les agents de glissement, les tensioactifs, les solubilisants, les agents mouillants ou leurs mélanges.

11. - Composition de comprimé sécable selon l'une quelconque des revendications précédentes, dans laquelle la composition comprend :
- 10-80% de fumarate de ténofovir disoproxil,
- 5-70% d'emtricitabine,
- 0,1-10% d'hydroxypropyl cellulose,
- 1-50% de lactose monohydraté,
- 0,1-50% de cellulose microcristalline,
- 1-50% de croscarmellose sodique,
- moins de 2 % de stéarate de magnésium,
- moins de 2 % de dioxyde de silicium colloïdal,
- moins de 1 % de lauryl sulfate de sodium en poids du premier composant (1)
- 10-95% d'éfavirenz,
- 0,1-10% d'hydroxypropyl cellulose,
- 1-50% de lactose monohydraté
- 0,1-50% de cellulose microcristalline,
- 0,5-50% de croscarmellose sodique,
- moins de 2% de stéarate de magnésium,
- moins de 2 % de dioxyde de silicium colloïdal,
- 2-10% de laury lsulfate de sodium en poids du second composant (2), et
- 1-10% d'enrobage pelliculaire en poids de la composition totale.

12. - Procédé de préparation de la composition de comprimé sécable selon la revendication 11, comprenant les étapes suivantes :
- préparation du mélange composant le premier composant (1) par granulation par voie humide
- préparation du mélange composant le second composant (2) par granulation par voie humide
- compression de ces deux mélanges totaux ensemble en comprimés bicouches sécables
- enrobage de ces comprimés bicouches sécables par une solution d'enrobage pelliculaire.

13. - Procédé selon la revendication 12, dans lequel ledit mélange composant le premier composant (1) est préparé en suivant ces étapes :
- mélange du fumarate de ténofovir disoproxil, de l'emtricitabine, de la cellulose microcristalline, du lactose monohydraté, de l'hydroxypropyl cellulose et d'un tiers de la croscarmellose sodique
- granulation de ce mélange avec de l'eau
- tamisage des granulés et leur séchage
- tamisage à nouveau des granulés séchés
- addition des deux-tiers de la croscarmellose sodique, du lauryl sulfate de sodium et de dioxyde de silicium colloïdal aux granulés tamisés et leur mélange
- addition du stéarate de magnésium et son mélange.

14. - Procédé selon la revendication 13, dans lequel ledit mélange composant le second composant (2) est préparé en suivant ces étapes :
- mélange de l'éfavirenz, de la cellulose microcristalline, du lactose monohydraté, de l'hydroxypropyl cellulose et de la croscarmellose sodique
- granulation de ce mélange avec de l'eau et du lauryl sulfate de sodium
- tamisage des granulés et leur séchage
- tamisage à nouveau des granulés séchés
- addition du dioxyde de silicium colloïdal aux granulés tamisés et leur mélange
- addition du stéarate de magnésium et son mélange.
